# EUROPÄISCHE PATENTSCHRIFT

(11) **EP 0 562 445 B1**
(45) Veröffentlichungstag und Bekanntmachung des Hinweises auf die Patenterteilung: **24.11.1999**
(21) Anmeldenummer: 93104378.0
(22) Anmeldetag: 17.03.1993
(51) Int. Cl.: A61K 47/32, A61K 47/36, A61K 9/06

(54) **Gel, insbesondere für die Augenheilkunde**
Ophthalmic gel
Gel opththalmique

(30) Priorität: 25.03.1992 DE 4209722
(43) Veröffentlichungstag der Anmeldung: 29.09.1993
(73) Patentinhaber: MEDPROJECT PHARMA-ENTWICKLUNGS- UND VERTRIEBSGESELLSCHAFT mbH, 82054 Sauerlach (DE)
(72) Erfinder: Löbering, Hans-Georg, Dr., W-8000 München 90 (DE); Polzer, Heinz, Dr., W-8028 Taufkirchen (DE)
(74) Vertreter: von Füner, Alexander, Prof.h.c. Dr.

(56) Entgegenhaltungen:
- EP-A- 0 056 420
- EP-A- 0 275 054
- EP-A- 0 312 208
- WO-A-84/04680
- WO-A-92/00707
- FR-A- 2 333 500
- CHEMICAL ABSTRACTS, vol. 115, no. 16, 21. Oktober 1991, Columbus, Ohio, US; abstract no. 166530r, N. M. DAVIES ET AL 'Evaluation of mucoadhesive polymers in ocular drug delivery. I. Viscous solutions'

## Beschreibung

Die Erfindung betrifft neue Arzneimittel, und zwar Gele
zur Anwendung am Auge. Üblicherweise werden Wirkstoffe am Auge in Form von Salben, Tropfen, Inserts oder auch Gelen zugeführt, wobei alle diese Anwendungsarten ihre Vor- und Nachteile haben. Die für das Auge angenehmste Art der Behandlung ist immer noch der wässrige Tropfen. Gegenüber den anderen genannten Formen hat er jedoch die geringste Verweildauer am Auge, so daß die Behandlungsfrequenz entsprechend häufiger und die systemische Belastung damit größer wird.

Insbesondere die Entwicklung von Gelen, die eine längere Verweildauer auf der Augenoberfläche aufweisen, tritt daher immer mehr in den Vordergrund. Häufig haben diese Gele salbenartige Konsistenz, und werden deshalb in Tuben abgefüllt. Die Handhabung von Tuben zur Applikation eines Tropfens am Auge hat Nachteile in vielerlei Hinsicht, weshalb Ärzte und Patienten Augenmedikamente in Kunststoffflaschen bevorzugen. Es fehlt bis heute ein Produkt, bei welchem die Vorteile eines wässerigen Tropfens bezüglich Verträglichkeit und Handhabung sinnvoll kombiniert sind mit einer längeren Verweildauer am Auge. Obgleich einige Patente über Gele und Gelformulierungen existieren, wurden darin spezielle Erfordernisse für die Anwendung am Auge noch nicht herausgearbeitet.

Gele unter Verwendung von Carbomer und Mischungen von Carbomer mit anderen Polymeren sind bekannt. Insbesondere bei der Anwendung am Auge haben Carbomergele die Eigenschaft, ihre Konsistenz nach Applikation beträchtlich zu verändern. Damit ist ein erwünschter, wasserspendender Effekt verbunden, wodurch dieses Gel, insbesondere bei trockenem Auge, Vorteile hat. Um jedoch gleichzeitig eine gegenüber rein wässerigen Tropfen längere Verweildauer zu erzielen, muß die Carbomerkonzentration so hoch sein, daß das Gel relativ hochviskos und damit nur aus Tuben entnehmbar und deshalb unvorteilhaft bei der Anwendung ist.

Mischungen von Carbomer mit anderen Polymeren zur Herstellung von Gelen wurden bisher in erster Linie für dermatologische Zwecke, als "Haftsalben"und in Form von "Gelpflastern" zur Wundabdeckung (z.B. US-Patent 4524064, US-Patent 5013769, EP 280 737 A 1) entwickelt. obgleich auch hier in einer Vorstufe Gele hergestellt werden mußten, verfolgte man mit den Produkten ganz andere Ziele, nämlich die Abdeckung von Wunden. Dazu müssen sie relativ schwer wasserlöslich und elastisch sein, sollten außerdem eine geringe Schrumpfung und eine starke Adhäsion auf der Haut zeigen. Bei dieser Art der Anwendung ist die Aufnahme von Wundsekret besonders wichtig. Dies wird in den Gelen durch einen hohen Einsatz von wasserbindenden Mitteln wie Sorbitol, Mannitol o.ä. erzielt. Am Auge ist eine wesentlich über die isotonische Konzentration hinausgehende Menge an Sorbit o.ä. schädlich. Zwangsläufig ergeben sich daher für die in den oben genannten Patenten beanspruchten Formulierungen ganz andere, für die Anwendung am Auge eher ungünstige Eigenschaften, beispielsweise Feuchtigkeitsentzug und schlechte Verträglichkeit aufgrund des hohen Materialeinsatzes. Ein ähnlicher Effekt ist von Augeninserts bekannt.

Ein anderes Patent (US 4983 385) beschäftigt sich mit der Verbesserung der Haftfähigkeit von fetthaltigen Salben insbesondere auf feuchten Körperflächen, wie z.B. Schleimhäuten. Die Problemlösung erfolgte durch Einarbeitung von Carbomer und anderen wasserlöslichen Polymermischungen in die Wasserphase der Salbe.

Ein weiteres Patent (EP 312 208 A 1) beschreibt zwar wässerige Gele, allerdings mit jeweils nur einem Polymer. Auch hier werden jedoch spezielle Anforderungen für die Anwendung am Auge (z.B. bezüglich Verträglichkeit und Applikationsform) nicht berücksichtigt.

Die WO 92/00707 beschreibt eine ophthalmische, wässerige, gelartige Suspension zur Behandlung bei trockenem Auge, die Wasser, zwischen ungefähr 0,1 und ungefähr 6,5 Gew.-% (bezogen auf das Gesamtgewicht der Suspension) eines Carboxylgruppen tragenden mit nicht-Polyalkylen-polyethern vernetzten Polymers, und zwischen ungefähr 0,01 und ungefähr 4 Gew.-% (bezogen auf das Gesamtgewicht der Suspension) mindestens eines ophthalmischen Demulcens enthält, wobei die Suspension einen pH zwischen ungefähr 6,6 und ungefähr 8,0 und eine Viskosität zwischen ungefähr 500 und ungefähr 4000 centipoises aufweist. In dieser Suspension kann Polyvinylalkohol und/oder Dextran 70 als Demulcens und bevorzugt Carbopol 976 als Polymer eingesetzt werden. Demulcens und vernetztes Polymer liegen in der Suspension in einem Mengenverhältnis zwischen 0,012:1 und 0,57:1 vor.

Aufgabe der Erfindung ist es, Gele zur Anwendung am Auge zur Verfügung zu stellen, die folgende Eigenschaften besitzen:
- die Gele sind tropfbar und aus handelsüblichen Augentropfenfläschchen entnehmbar.
- Sie weisen eine einstellbare Restviskosität am Auge auf, die maßgeblich für Verträglichkeit und Verweildauer verantwortlich ist.
- Alle üblicherweise in der Ophthalmologie verwendeten Wirkstoffe und Konservierungsmittel können in die Rezeptur inkorporiert werden.

Die hier zusammengefaßten Eigenschaften werden nachfolgend detailliert beschrieben:
1. Die Gele sind tropfbar: Für die Tropfbarkeit ist die Ausgangsviskosität ausschlaggebend. Die Tropfbarkeit gewährleistet eine bessere Handhabung, da das Produkt aus einer handelsüblichen Augentropfflasche angewendet werden kann. Die Dosierungsgenauigkeit ist aus Flaschen wesentlich besser als bei Gelen mit dem aus Tuben üblicherweise kommenden "Strang". Geeignete Ausgangsviskositäten liegen im Bereich von 10 000 - 50 000 mPas (Viskositätswerte beziehen sich auf die in den Beispielen aufgeführte Meßmethode).
2. Am Auge stellt sich durch die Vermischung des Gels mit Bestandteilen des Tränenfilmes, in erster Linie mit den anorganischen Salzen, eine bestimmte Restviskosität ein. Diese Restviskosität ist aufgrund der bekannten Eigenschaften des Carbomers (Gelstruktur bricht zusammen) erheblich geringer als die Ausgangsviskosität vor dem Kontakt mit der Tränenflüssigkeit.
   Die sich am Auge einstellende Restviskosität ist letztendlich ausschlaggebend für Verträglichkeit und Verweildauer des Gels auf der Augenoberfläche. Durch steigende Restviskosität werden ebenso Wirkungsdauer und Wirkungsintensität des Augenmedikamentes positiv beeinflußt. Eine zu hohe Restviskosität führt hingegen zu Klebstoffeffekten, Fremdkörpergefühl, also zu Verträglichkeitsproblemen. Geeignete Restviskositäten liegen im Bereich von 20 - 2000 mPas (Viskositäten beziehen sich auf die in den Beispielen aufgeführten Meßmethoden).
3. Der Einarbeitung von Wirkstoffen und Konservierungsmitteln in Carbomergele sind dadurch Grenzen gesetzt, daß es gelegentlich durch Interaktion mit den Carboxylgruppen des Carbomers, insbesondere mit organischen Basen, zu Ausfällungen kommen kann. Der überwiegende Anteil der in der Ophthalmologie verwendeten Wirkstoffe besteht aus organischen Basen oder deren Salzen. Auch einige wichtige Konservierungsmittel gehören zu diesem Typ.

Die oben gestellte Aufgabe wird wie aus den nachstehenden Ansprüchen ersichtlich gelöst.

Die erfindungsgemäße Lösung für die Formulierung eines Gels mit den in den 3 Punkten genannten Eigenschaften läßt sich wie folgt beschreiben:

Um aus der Kombination der unter den Punkten 1) und 2) aufgeführten Eigenschaften eine sinnvolle und zweckmäßige Rezeptur zu erstellen, galt es, zwei sich gleichsinnig ändernde Effekte zu beeinflussen, nämlich Ausgangs- und Restviskosität.

Die Tropfbarkeit des Gels erfordert eine relativ niedrige Ausgangsviskosität und damit einen relativ geringen Materialeinsatz des Gelbildners. Daraus ergibt sich in der Regel nach Applikation am Auge auch eine niedrige Restviskosität, die dann kaum zu einer merklichen Verbesserung der Verweildauer des Gels am Auge führt. Erhöht man den Materialeinsatz des Gelbildners (Carbomer) zur Erzielung einer geeigneteren (höheren) Restviskosität, so resultiert daraus eine Ausgangsviskosität, die in der Praxis eine Tropfbarkeit aus Flaschen nicht mehr erlaubt. Durch gezielte Salzzugabe (anorganische Salze oder auch Salze organischer Amine und Aminosäuren) zu diesen Gelen mit hoher Ausgangsviskosität kann Jedoch die Ausgangsviskosität reduziert werden bis zu einem Punkt, bei dem eine zufriedenstellende Tropfbarkeit erreicht wird.

Eine Aufgabe der Erfindung beinhaltet somit die Entwicklung einer Rezeptur, bei der ein definiertes Verhältnis von Ausgangs- und Restviskosität einstellbar ist, mit dem Ziel, ein tropfbares Gel mit verlängerter Verweildauer auf der Augenoberfläche herzustellen. Da jedoch zusätzlich die unter Punkt 3) genannte Einarbeitung von Wirkstoffen und ggf. Konservierungsmitteln mitberücksichtigt werden muß, konnte dies nicht allein durch Herausfinden einer geeigneten Carbomerkonzentration und anschließender Salzzugabe, also durch Ausnutzen bekannter Carbomereigenschaften, erreicht werden, sondern es mußte eine geeignete neue Formulierung gefunden werden. Als geeignet hinsichtlich Formulierbarkeit und guter Verträglichkeit am Auge erwies sich ein Gemisch von Carbomer/ Polyvinylalkohol(PVA), Carbomer/ Dextran, oder Carbomer/ PVA/Dextran. Die Mischung aus Carbomer und PVA erweist sich als besonders vorteilhaft, da PVA nicht nur lösungsvermittelnde, sondern zusätzlich auch filmbildende Eigenschaften besitzt. Dies ist ein besonders bei der Anwendung am Auge erwünschter Effekt. Das Gemisch von Carbomer und PVA bietet somit auch in wirkstofffreier Form Vorteile gegenüber reinen Carbomergelen .

Überraschend und wider Erwarten stellte sich heraus, daß es schwierig ist, stabile Gele aus Carbomer und PVA herzustellen. Da Gele zur Anwendung am Auge steril sein müssen, ist die Autoklavierbarkeit des Fertigproduktes zwingend notwendig. An die Temperaturstabilität dieser Gele sind daher besonders hohe Ansprüche zu stellen. Dies gilt natürlich auch bezüglich der erforderlichen Haltbarkeit des Fertigproduktes im Endbehältnis über die Produktlaufzeit. Die Streßbedingung einer Temperaturbelastung gibt gleichzeitig auch einen Hinweis auf die Haltbarkeit und Langzeitstabilität. Erfindungsgemäß wurde gefunden, daß nicht jedes beliebige Mischungsverhältnis von Carbomer und PVA zu in ihren allgemeinen Merkmalen, insbesondere der Ausgangsviskosität, stabilen Produkten führt (siehe Tabelle 1).

Erfindungsgemäß können alle marktüblichen Carbomertypen (Polyacrylattypen) eingesetzt werden. Zweckmäßigerweise wählt man wasserlösliche Typen mit einem Molekulargewicht zwischen 1.000.000 und 4.000.000, im konkreten Fall waren dies Carbopol 934, 934P, 940, 941, 951, 954, 974, 974P, 980, 981 (siehe auch H.P. Fiedler, Lexikon der Hilfsstoffe für Pharmazie, Kosmetik und angrenzende Gebiete, 3. Auflage 1989, Edition Cantor Aulendorf, Stichworte, Polyacrylsäure, Carbopol).

Bei PVA führen jedoch nur vollständig verseifte Typen (d.h. Hydrolysegrad von mindestens 99%, Molekulargewicht 15 000 bis 200 000) im richtigen Mischungsverhältnis mit Carbomer zu viskositäts- und pH-stabilen Gelen. Bei konstanter Carbomermenge wird das Produkt mit steigendem PVA-Anteil bezüglich der Ausgangsviskosität instabil. Die nachfolgende Tabelle 1 gibt dies wieder.

Es stellte sich heraus, daß das Verhältnis von PVA : Carbomer nicht mehr als 8 : 1 betragen darf.

Tabelle 2 zeigt Beispiele von Rezepturen, woraus Einflüsse der Zusammensetzung, insbesondere des Mischungsverhältnisses PVA : Carbomer, auf Ausgangs- und Restviskosität erkennbar sind:

**Tabelle 1**

| Abhängigkeit der Stabilität der Gele vom Verhältnis Carbomer/PVA (Viskositätsvergleich vor und nach Autoklavieren) | | | |
|---|---|---|---|
| | Viskosität (20°C,1,5 rpm, Sp 63) | | |
| Formulierung | vor Autoklavieren | nach Autoklavieren | % Änderung |
| 0,25% Carbomer | 16.700 | 15.500 | - 7,2 |
| 0,5% PVA | | | |
| 0,07% Na-acetat | | | |
| 0,25% Carbomer | 38.400 | 32.600 | - 15 |
| 0,5% PVA | | | |
| 0,04% Na-acetat | | | |
| 0,25% Carbomer | 22.100 | 21.400 | - 5,7 |
| 0,6% PVA | | | |
| 0,065% Na-acetat | | | |
| 0,25% Carbomer | 25.400 | 22.400 | - 12 |
| 0,7% PVA | | | |
| 0,060% Na-acetat | | | |
| 0,25% Carbomer | 27.200 | 23.000 | - 15 |
| 0,8% PVA | | | |
| 0,055% Na-acetat | | | |
| 0,25% Carbomer | 24.700 | 20.900 | - 15,4 |
| 1% PVA | | | |
| 0,035% Na-acetat | | | |
| 0,25% Carbomer | 20.600 | 11.800 | - 42,7 |
| 2% PVA | | | |
| 0,064% Na-acetat | | | |
| 0,5% Carbomer | 36.300 | 38.400 | + 5,8 |
| 0,5% PVA | | | |
| 0,27% Na-acetat | | | |
| 0,5% Carbomer | 29.400 | 2.700 | - 8,1 |
| 1% PVA | | | |
| 0,28% Na-acetat | | | |
| 0,5% Carbomer | 38.000 | 21.500 | - 43,4 |
| 4% PVA | | | |
| 0,2% Na-acetat | | | |
| 1% Carbomer | 30.200 | 28.100 | - 6,9 |
| 1% PVA | | | |
| 1,9% Na-acetat | | | |

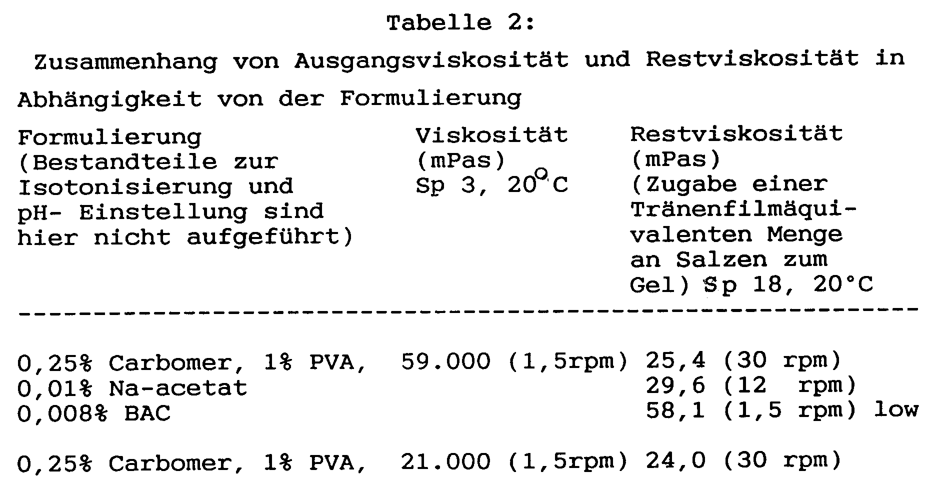

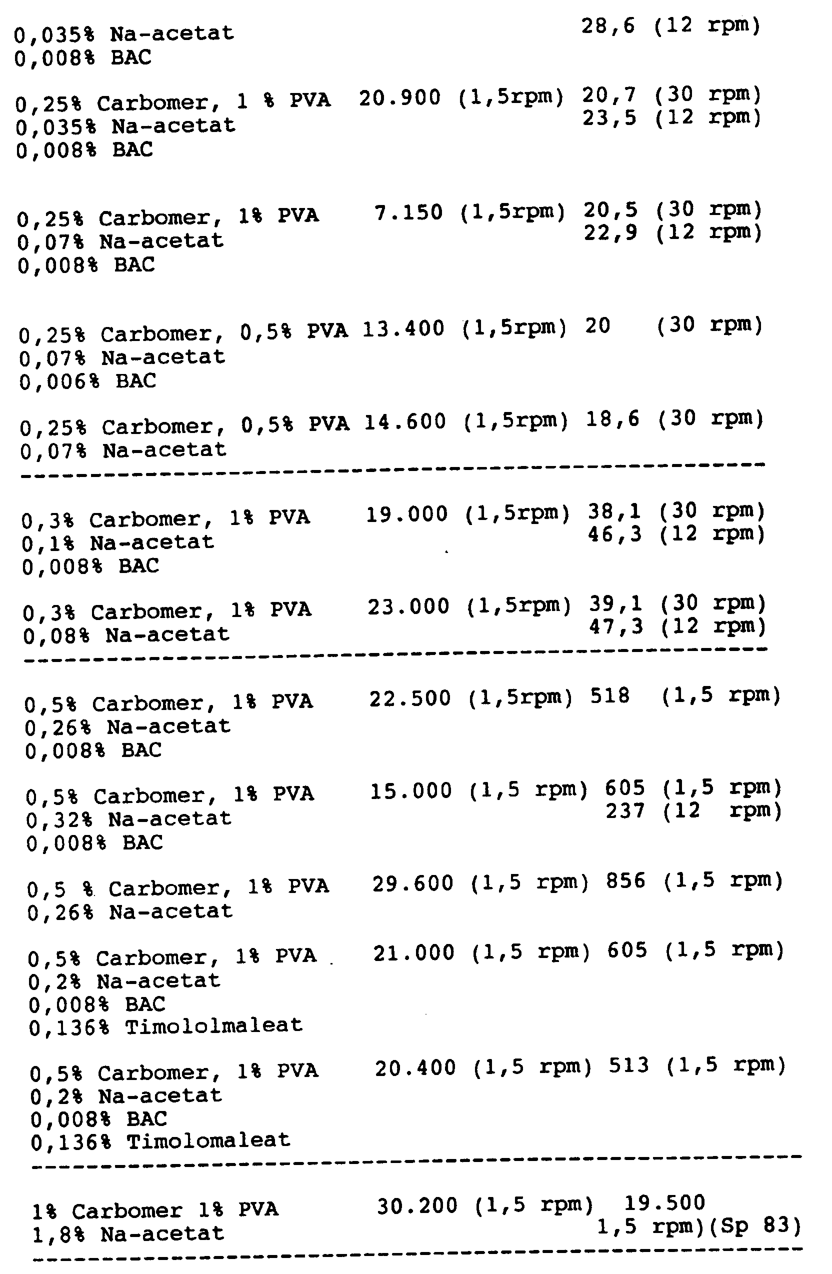

Folgerungen aus Tabelle 2:
- Die sich ergebene Restviskosität ist praktisch nur von der eingesetzten Carbomermenge abhängig. Sie ist weitgehend unabhängig von der Ausgangsviskosität sowie von der Menge an PVA.
- Die Restviskosität steigt mit der Carbomermenge drastisch an und ist bei 1% Carbomergehalt bereits in einem Bereich, der für die Verträglichkeit am Auge nicht mehr akzeptabel ist.
- Die Ausgangsviskosität ist abhängig von der Salzkonzentration und der Carbomerkonzentration, d.h. bei gleichem Carbomergehalt erniedrigt sie sich mit steigender Salzkonzentration. Bei steigender Carbomerkonzenzentration muß mehr Salz zugegeben werden, um gleiche Ausgangsviskositäten zu erhalten.

Da für die Bildung von Polyacrylatgelen der Zusatz von Basen, in der Regel NaOH, erforderlich ist, müssen die erfindungsgemäß beanspruchten Gele ebenfalls Basen enthalten. Besonders gute Resultate ergeben sich in unserem Fall mit der Aminosäure Lysin als Base.

Neben Lysin eignen sich auch noch andere basische Aminosäuren, z.B. Ornithin, α,ω-Diaminobuttersäure, Arginin, Histidin oder auch Hydroxylysin (einem Bestandteil des natürlichen Kollagens). Prinzipiell ist die Gelbildung mit allen Basen möglich. Für handelsübliche Augengele wird NaOH benutzt. Bevorzugt wird Lysin gewählt, weil das Gel nach Anwendung auf der Haut sich "zügiger" anfühlt und sehr gut verträglich ist. Die Gelbildung durch Amine ist ebenfalls möglich.

Da Gele zur Anwendung am Auge üblicherweise isoton sein sollten, müssen diese Gele mit geeigneten Mitteln isotonisiert werden. In der Regel werden hierfür Sorbitol oder Mannitol verwendet. Es kommen hierfür nur nichtionische Verbindungen in Betracht. Glycerol kann ebenfalls eingesetzt werden Das Auge toleriert Osmolaritäten zwischen 100 und 450 mOsmol/l. In der Regel werden Augentropfen mit einem Isotonans etwa auf 280 bis 320 mOsmol/1 eingestellt, und zwar mit Salzen. Salze (Ionen) können für Carbomer in den beanspruchten Konzentrationen aus den oben erwähnten Gründen nicht verwendet werden, weil sie die Gelstruktur zerstören.

Die Gele können ohne pharmakologisch wirksamen Bestandteil als Tränensubstitutionsmittel eingesetzt werden. Andererseits sind sie auch als Vehikel für Wirkstoffe geeignet. Es ist besonders schwierig, organische Basen (= Wirkstoffe) und Konservierungsmittel vom Quat-Typ in klare und stabile Polyacrylatgele einzuarbeiten. Erfindungsgemäß wurden Formulierungen und Verfahren gefunden, die dies ermöglichen.

Ein Beispiel für ein übliches Konservierungsmittel ist Benzalkoniumchlorid (BAC), wobei zu beachten ist, daß der Zusatz eines Konservierungsmittels für Augenarzneimittel in Mehrdosenbehältern vorgeschrieben ist. Carbomer und Benzalkoniumchlorid sind schlecht kompatibel. Der Zusatz eines zweiten, gut wasserlöslichen Polymers verhindert die störenden Effekte (darüber hinaus ermöglicht er auch die Einarbeitung von Wirkstoffen in gelöster Form). Andere Konservierungsmittel sind mit der erfindungsgemäßen Kombination ebenfalls kompatibel, z.B. die am Auge gut verträglichen Konservierungsmittel Thiomersal, Chlorhexidingluconat/-acetat, Parabene. BAC gilt als repräsentativ für die Gruppe der "Quats", auch für polymere, Quat-ähnliche Stoffe.

Die Erfindung betrifft also:
1. Die Herstellung tropfbarer Gele aus einem Polymergemisch von Polyacrylat/PVA, Polyacrylat/Dextran, Polyacrylat/PVA Dextran, unter Zugabe von Salzen zur Beeinflussung von Ausgangsviskosität und Restviskosität.
2. Die unter Punkt 1) genannten Gele können wirkstoffrei sein, oder auch Wirkstoffe enthalten. Als Wirkstoffe kommen alle in der Ophthalmologie üblicherweise eingesetzten Wirkstoffe in Betracht.
3. Die unter Punkt 1 und 2) genannten Gele können gegebenenfalls zusätzlich ein Konservierungsmittel enthalten, wobei hierfür alle in der Ophthalmologie üblicherweise eingesetzten Konservierungsstoffe in Betracht kommen.
4. Die unter Punkt 1), 2) und 3) genannten Gele können zusätzlich mit geeigneten Stoffen isotonisiert werden, wozu nichtionische Verbindungen, wie oben genannt, eingesetzt werden.
5. Zur Einstellung der Euhydrie und zur Ausbildung der Gelstruktur ist es erforderlich, zusätzlich mit einer Base zu neutralisieren. Als Basen sind NaOH, Amine oder auch Aminosäuren geeignet.

Die erfindungsgemäßen Gele werden wie folgt hergestellt: In einem temperierbaren und evakuierbaren Reaktor, ausgerüstet mit Rührer und Homogenisator wird in der Wärme eine wässerige Lösung des/der mit Carbomer zu kombinierenden Polymeren hergestellt. Dann werden darin Isotonans, Wirkstoff(e) und ggf. Konservierungsmittel und/oder andere übliche Zusatzstoffe gelöst. Im nächsten Schritt wird das Carbomer dispergiert und durch Zugabe von Base das Gel gebildet. Schließlich wird das Gel kurz homogenisiert und entlüftet (Homogenisierung und Entlüftung kann während des Abkühlens durchgeführt werden).

Die nachfolgenden Beispiele erläutern die Erfindung.

### Formulierungsbeispiele

Es handelt sich immer um Hydrogele (Basis Wasser)

### Beispiel 1

Zusammensetzung (in %):

| | |
|---|---|
| Polyvinylalkohol | 1 |
| Sorbitol | 4,5 |
| Carbomer (Polyacrylsäure) | 0,19 |
| DL-Lysin | 0,34 |
| Benzalkoniumchlorid | 0,008 |

Herstellung:
In einem evakuierbaren Reaktor mit Rührer und Homogenisator werden 13,5 g Sorbitol in 80 g Wasser gelöst, mit 75 g 4%iger Polyvinylalkohol-Lösung gemischt und auf 70 bis 80°C erwärmt. Bei laufendem Homogenisator gibt man 0,57 g Carbomer in kleinen Anteilen zur Lösung und läßt den Homogenisator nach beendeter Zugabe zur vollständigen Dispergierung noch 5 min weiterlaufen. Zu diesem Gemisch werden unter Rühren langsam 100 g einer 0,02%igen Lösung von Benzalkoniumchlorid getropft. Das Gel entsteht durch Zugabe von 31 g einer 3,4%igen Lösung von Lysin. Während des Abkühlens wird der Reaktor zur Entlüftung des Gels evakuiert.

Viskosität (Brookfield-Viskosimeter):
38.700 mPas nach Autoklavieren (Spindel 3, 1,5 U/min, 20°C) pH 7,7.

### Beispiel 2

Zusammensetzung (in %):

| | |
|---|---|
| Polyvinylalkohol | 1 |
| Sorbitol | 4,5 |
| Natriumacetat, wasserfrei | 0,04 |
| Carbomer (Polyacrylsäure) | 0,25 |
| DL-Lysin | 0,43 |
| Benzalkoniumchlorid | 0,008 |

Herstellung:
In einem evakuierbaren Reaktor mit Rührer und Homogenisator werden 13,5 g Sorbitol und 0,12 g Natriumacetat in 180 g Wasser gelöst, mit 75 g 4%iger Polyvinylalkohol-Lösung gemischt und auf 70 bis 80°C erwärmt. Bei laufendem Homogenisator gibt man 0,57 g Carbomer in kleinen Anteilen zur Lösung und läßt den Homogenisator nach beendeter Zugabe zur vollständigen Dispergierung noch 5 min weiterlaufen. Das Gel entsteht durch Zugabe von 16,3 g einer 7,9%igen Lösung von Lysin. Zum Gel werden unter Rühren langsam 14,5 ml einer 0,17%igen Lösung von Benzalkoniumchlorid getropft. Während des Abkühlens wird der Reaktor zur Entlüftung des Gels evakuiezt.

Viskosität (Brookfield-Viskosimeter):
16.900 mPas nach Autoklavieren (Spindel 3, 1,5 U/min, 20°C) pH 7,6

### Beispiel 3

Zusammensetzung (in %):

| | |
|---|---|
| Polyvinylalkohol | 1 |
| Sorbitol | 4,5 |
| Natriumacetat, wasserfrei | 0,14 |
| Carbomer (Polyacrylsäure) | 0,5 |
| DL-Lysin | 0,89 |
| Benzalkoniumchlorid | 0,008 |

Herstellung wie Beispiel 2.

Viskosität (Brookfield-Viskosimeter):
36.800 mPas nach Autoklavieren (Spindel 3, 1,5 U/min, 20°C) pH 7,2

### Beispiel 4

Zusammensetzung (in %):

| | |
|---|---|
| Dextran 70 | 2,0 |
| Sorbitol | 4,8 |
| Carbomer (Polyacrylsäure) | 0,16 |
| DL-Lysin | 0,27 |
| Benzalkoniumchlorid | 0,008 |

Herstellung:
In einem evakuierbaren Reaktor mit Rührer und Homogenisator werden 19,2 g Sorbitol und 8 g Dextran in 221 g Wasser bei 40 bis 60°C gelöst. Bei laufendem Homogenisator gibt man 0,64 g Carbomer in kleinen Anteilen zur Lösung und läßt den Homogenisator nach beendeter Zugabe zur vollständigen Dispergierung noch 5 min weiterlaufen. Zu diesem Gemisch werden unter Rühren langsam 100 g einer 0,032%igen Lösung von Benzalkoniumchlorid getropft. Das Gel entsteht durch Zugabe von 51 g einer 2,1%igen Lösung von Lysin. Während des Abkühlens wird der Reaktor zur Entlüftung des Gels evakuiert.

Viskosität (Brookfield-Viskosimeter):
30.200 mPas nach Autoklavieren (Spindel 3, 1,5 U/min, 20°C) pH 7,4

### Beispiel 5

Zusammensetzung (in %):

| | |
|---|---|
| Polyvinylalkohol | 1 |
| Sorbitol | 4,5 |
| Timololhydrogenmaleat | 0,5 |
| Carbomer (Polyacrylsäure) | 0,5 |
| DL-Lysin | 1,1 |
| Benzalkoniumchlorid | 0,008 |

Herstellung:
In einem evakuierbaren Reaktor mit Rührer und Homogenisator werden 13,5 g Sorbitol in 80 g Wasser gelöst, mit 75 g 4%iger Polyvinylalkohol-Lösung gemischt und auf 70 bis 80°C erwärmt. Man löst 1,5 g Timololhydrogenmaleat in dem Gemisch, gibt bei laufendem Homogenisator 1,5 g Carbomer in kleinen Anteilen zur Lösung und läßt den Homogenisator nach beendeter Zugabe zur vollständigen Dispergierung noch 5 min weiterlaufen. Zu diesem Gemisch werden unter Rühren langsam 100 g einer 0,024%igen Lösung von Benzalkoniumchlorid getropft. Das Gel entsteht durch Zugabe von 28,3 g einer 11,7%igen Lösung von Lysin. Während des Abkühlens wird der Reaktor zur Entlüftung des Gels evakuiert.

Viskosität (Brookfield-Viskosimeter):
33.200 mPas nach Autoklavieren (Spindel 3, 1,5 U/min, 20°C) pH 7,4

### Beispiel 6

Zusammensetzung (in %):

| | |
|---|---|
| Polyvinylalkohol | 1 |
| Mannitol | 5 |
| Carbomer (Polyacrylsäure) | 0,165 |
| DL-Lysin | 0,25 |
| Benzalkoniumchlorid | 0,008 |
| Prednisolonacetat | 0,5 |

Herstellung:
In einem evakuierbaren Reaktor mit Rührer und Homogenisator werden 15 g Mannitol in 170 g Wasser gelöst, mit 75 g 4%iger Polyvinylalkohol-Lösung gemischt und auf 70 bis 80°C erwärmt.

Bei laufendem Homogenisator gibt man 0,495 g Carbomer in kleinen Anteilen zur Lösung und läßt den Homogenisator nach beendeter Zugabe zur vollständigen Dispergierung noch 5 min weiterlaufen. Das Gel entsteht durch Zugabe von 15,75 g einer 4,76%igen Lösung von Lysin. Während des Abkühlens wird der Reaktor zur Entlüftung des Gels evakuiert.

In 23 g Wasser, das 0,15 ml 16,4%ige BenzalkoniumchloridLösung enthält, werden mit einem Homogenisator 1,5 g Prednisolonacetat fein verteilt. Die entstandene Suspension wird in kleinen Anteilen zum Gel gegeben. Nach beendeter Zugabe läßt man den Rührer zur Homogenisierung noch 1 Stunde weiterlaufen.

Viskosität (Brookfield-Viskosimeter):
35.700 mPas nach Autoklavieren (Spindel 3, 1,5 U/min, 20°C) pH 7,0

## Patentansprüche

1. Tropfbares Gel zur Anwendung am Auge, das eine Viskosität von 10.000 bis 50.000 mPas, gemessen mit einem Brookfield-Viskosimeter, aufweist, was erreicht wird durch die Zusammensetzung, enthaltend
1) ein Polyacrylat in einer Menge von 0,05 bis 3%,
2) ein wasserlösliches physiologisch verträgliches Polymer oder Polymergemisch, nämlich Polyvinylalkohol und/oder Dextran in einer Menge von 0,1 bis 10%,
3) die für Gele zur Anwendung am Auge üblichen Hilfsstoffe,
4) ein Salz zur Einstellung der Viskosität und
5) eine Base,
wobei das Verhältnis von Komponente 2) zu Komponente 1) zwischen 1:1 und 8:1 liegt.

2. Gel nach Anspruch 1, dadurch **gekennzeichnet**, daß es einen oder mehrere Wirkstoffe aus der Gruppe der in der Ophthalmologie bekannten enthält.

3. Gel nach Anspruch 1 oder 2, dadurch **gekennzeichnet,** daß das Verhältnis von Komponente 2) zu Komponente 1) zwischen 2:1 und 5:1 liegt.

4. Gel nach einem der Ansprüche 1 bis 3, dadurch **gekennzeichnet**, daß es zur Einstellung der Spanne zwischen Ausgangsviskosität und Restviskosität als Salz Natriumacetat in einer Menge von 0,01 bis 0,5% enthält.

5. Gel nach einem der Ansprüche 1 bis 4, dadurch **gekennzeichnet**, daß es als Polyacrylat Typen mit einem Molekulargewicht zwischen 1.000.000 und 4.000.000 enthält.

6. Gel nach einem der Ansprüche 1 bis 5, dadurch **gekennzeichnet**, daß es als Base Lysin enthält.

7. Gel nach einem der Ansprüche 1 bis 6, dadurch **gekennzeichnet**, daß es als isotonisierendes Mittel zusätzlich Sorbitol oder Mannitol enthält.

8. Gel nach einem der vorangegangenen Ansprüche, dadurch **gekennzeichnet**, daß es als Konservierungsmittel Benzalkoniumchlorid enthält.

## Claims

1. A drippable ophthalmologic gel having a viscosity in the range of 10,000 to 50,000 mPas as measured with a Brookfield viscometer, said gel being achieved by providing a composition comprising
(1) a polyacrylate in an amount of 0.05 to 3% by weight;
(2) a water-soluble, physiologically compatible polymer selected from the group consisting of polyvinyl alcohol, dextran, and mixtures thereof;
(3) adjuvants normally used in ophthalmologic gels;
(4) a salt for adjusting the viscosity; and
(5) a base;
wherein the ratio of component (2) to component (1) is in the range of 1:1 to 8:1.

2. A gel according to claim 1, **characterized** by having one or more active ingredients selected from those generally known in ophthalmology.

3. A gel according to claims 1 or 2, **characterized** in that the ratio of component (2) to component (1) is in the range of 2:1 to 5:1.

4. A gel according to any of claims 1 to 3, **characterized** by containing sodium acetate in an amount of 0.01 to 0.5% as a salt for the purpose of adjusting the difference between the original viscosity and the residual viscosity.

5. A gel according to any of claims 1 to 4, **characterized** by containing as the polyacrylate such types having a molecular weight between 1,000,000 and 4,000,000.

6. A gel according to any of the claims 1 to 5, **characterized** by containing lysine as a base.

7. A gel according to any of the claims 1 to 6, **characterized** by further comprising sorbitol or mannitol as an isotonizing agent.

8. A gel according to any of the preceding claims, **characterized** by comprising benzalkonium chloride as a preservative.

## Revendications

1. Gel fluide destiné à être utilisé au niveau de l'oeil, présentant une viscosité de 10.000 à 50.000 mPas, mesurée avec un viscosimètre de Brookfield, et obtenu grâce à la composition suivante, qui contient
1) un polyacrylate en quantité allant de 0,05 à 3%,
2) un polymère ou un mélange de polymères physiologiquement compatible et soluble dans l'eau, notamment de l'alcool polyvinylique et/ou du dextran, en quantité allant de 0,1 à 10%,
3) les auxiliaires habituels pour les gels utilisés au niveau de l'oeil,
4) un sel pour ajuster la viscosité et
5) une base,
le rapport entre le composant 2) et le composant 1) étant situé entre 1:1 et 8:1.

2. Gel selon la revendication 1, caractérisé en ce qu'il contient un ou plusieurs agents du groupe des agents connus en ophtalmologie.

3. Gel selon l'une des revendications 1 ou 2, caractérisé en ce que le rapport entre le composant 2) et le composant 1) est situé entre 2:1 et 5:1.

4. Gel selon l'une des revendications 1 à 3, caractérisé en ce qu'il contient, en tant que sel pour ajuster l'écart entre la viscosité de départ et la viscosité résiduelle, de l'acétate de sodium en quantité allant de 0,01 à 0,5%.

5. Gel selon l'une des revendications 1 à 4, caractérisé en ce qu'il contient, en tant que polyacrylate, des types de polyacrylate dont le poids moléculaire est compris entre 1.000.000 et 4.000.000.

6. Gel selon l'une des revendications 1 à 5, caractérisé en ce qu'il contient, en tant que base, de la lysine.

7. Gel selon l'une des revendications 1 à 6, caractérisé en ce qu'il contient en plus, en tant qu'agent isotonisant, du sorbitol ou du mannitol.

8. Gel selon l'une des revendications précédentes, caractérisé en ce qu'il contient, en tant qu'agent conservateur, du chlorure de benzalkonium.
